Europäisches Patentamt

European Patent Office

Office européen des brevets

(19)

(11) Publication number: **0 212 927 B1**

# EUROPEAN PATENT SPECIFICATION

(12)

(45) Date of publication of patent specification: **23.10.91**

(51) Int. Cl.5: **A61K 31/35**, A61K 45/06, //(A61K31/35,31:195)

(21) Application number: **86306146.1**

(22) Date of filing: **08.08.86**

The file contains technical information submitted after the application was filed and not included in this specification

(54) **Pharmaceutical composition comprising sodium cromoglycate and a mucolytic agent.**

(30) Priority: **10.08.85 GB 8520133**

(43) Date of publication of application:
**04.03.87 Bulletin 87/10**

(45) Publication of the grant of the patent:
**23.10.91 Bulletin 91/43**

(84) Designated Contracting States:
**BE CH DE FR GB IT LI LU NL SE**

(56) References cited:

CHEMICAL ABSTRACTS, vol. 102, no. 4, 28th January 1985, page 466, abstract no. 32031b, Columbus, Ohio, US; L.J. LESKO et al.: "Physical-chemical compatibility of cromolyn sodium nebulizer solution-bronchodilator inhalant solution admixtures", & ANN. ALLERGY 1984, 53(3), 236-8

DICTIONNAIRE VIDAL, 1980, 56th edition, pages 702,703,866, OVP, Paris, FR: Pages 702,703: "Lomusol"; page 866: "Opticron collyre"

UNLISTED DRUGS, vol. 19, no. 11, November 1967, page 150, Chatham, New Jersey, US

PHARMAZEUTISCHE STOFFLISTE, 7th Edition, pages 370, 371

(73) Proprietor: **FISONS plc**
**Fison House Princes Street**
**Ipswich Suffolk IP1 1QH(GB)**

(72) Inventor: **Chawla, Brindra Paul Singh**
**45 Boundary Road**
**West Bridgford Nottingham(GB)**

(74) Representative: **Craig, Christopher Bradberry et al**
**Fisons plc 12 Derby Road**
**Loughborough Leicestershire LE11 0BB(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to pharmaceutical compositions comprising sodium cromoglycate and a mucolytic agent.

Sodium cromoglycate, the sodium salt of 1,3-bis(2-carboxychromon-5-yloxy)propan-2-ol, has been known for many years to be useful in the treatment of allergic conditions, notably of the lung but also of the nose and eye. Mucolytic agents, e.g. N-acetyl-1-cysteine, have been used to facilitate the removal of mucus in broncho-pulmonary disease. Certain mucolytic agents, in particular N-acetyl-1-cysteine, to achieve maximum therapeutic effect are administered by nebulisation of a 10-20% w/v aqueous solution having a pH of 7-9. This combination of relatively high pH and high concentration is incompatible with therapeutically effective amounts of sodium cromoglycate, which is known to degrade at such pH and tends to precipitate from solution in the presence of additional solutes.

We have now found that stable aqueous solutions containing a therapeutically effective amount of sodium cromoglycate and a mucolytic agent can be prepared.

According to the invention there is provided a pharmaceutical composition comprising an aqueous solution of from 0.6 to 6% w/v sodium cromoglycate and a mucolytic agent.

The composition preferably contains from 0.75 to 5% w/v and more preferably from 0.8 to 4% w/v of sodium cromoglycate, measured as the hydrated powder.

The composition is preferably in the form of a clear aqueous solution.

Suitable mucolytic agents include 4[[(2-amino-3,5-dibromophenyl)methyl)amino]cyclohexanol, 2-amino-3,5-dibromo-N-cyclohexylbenzenemethanamine and pharmaceutically acceptable, e.g. hydrochloride, salts thereof. However, we particularly prefer the mucolytic agent to be N-acetyl-1-cysteine or a pharmaceutically acceptable, e.g. sodium, salt thereof.

When the mucolytic agent is N-acetyl-1-cysteine we prefer the solution to contain from 1 to 9% w/v, more preferably 3 to 8% w/v, particularly 3.5 to 6.5% w/v, e.g. 5% w/v N-acetyl-1-cysteine, measured as the free acid.

Compositions for administration to the lung by nebuliser may be prepared without any additional excipients. However, certain aqueous formulations for administration, e.g. to the eyes, are preferably substantially clear, aqueous sterile solutions.

The composition may also contain an effective proportion of a pharmaceutically acceptable chelating or sequestering agent. Suitable sequestering or chelating agents include sodium carboxymethyl cellulose, citric, tartaric or phosphoric acid, and amino carboxylate compounds, preferably ethylenediamine tetraacetic acid or its salts, e.g. its calcium salt, its calcium-sodium salt, or more preferably its di-sodium salt.

The concentration of the chelating or sequestering agent may vary considerably, but in any case should be such as to ensure that no precipitate of metal salts of the cromoglycate anion occurs. A suitable concentration of chelating or sequestering agent may be from 0.005 to 0.1, and preferably from 0.01 to 0.1% w/v. When a very low concentration, i.e. less than 0.40, preferably less than 0.32 ppm of 'metal ions' are present, for example when the solution contains less than 0.08 ppm of ionic iron and less than 0.25 ppm of ionic zinc, the chelating or sequestering agent may if desired be dispensed with. When a chelating or sequestering agent is used the concentration of 'metal ions' is preferably less than 10 ppm.

By the term 'metal ions' we mean ions of metals in groups IIa, IIb and IVb (also those of groups IIIa and IVa) of the periodic table and of the transition metals. Specific 'metal ions' which are detrimental, in excessive concentrations, i.e. above about 20 ppm, to the compositions of the invention are $Pb^{++}$, $Ca^{++}$, $Mg^{++}$ and in particular $Fe^{++}$, $Fe^{+++}$, and $Zn^{++}$ ions. We particularly prefer to keep the concentration of $Mg^{++}$ ions as low as possible, e.g. less than 2.0 ppm and preferably less 0.2 ppm in the dry active ingredient and less than about 0.8 ppm in the water used to make the solution.

The solution composition may if desired contain an effective proportion, e.g. from 0.001 to 0.10% w/v, of a pharmaceutically acceptable preservative or sterilising agent. One example of a suitable preservative is sodium 2-(ethyl mercuriothio) benzoate known generically as 'Thiomersal®', which may be present in the composition in from 0.001% to 0.05 and preferably from 0.005 to 0.02, e.g. about 0.01% w/v. Other suitable preservatives include pharmaceutically acceptable quaternary ammonium compounds, e.g. cetyl pyridinium chloride, tetradecyltrimethyl ammonium bromide known generically as 'Centrimide', benzyl dimethyl [2-[2-[p-(1,1,3,3-tetramethyl butyl)]phenoxy]ethoxy] ammonium chloride, known generically as 'Benzethonium chloride', and myristyl-γ-picolinium chloride, any one of which may be used at a concentration of from 0.002 to 0.05, e.g. about 0.02% w/v. The preferred preservatives amongst the quaternary ammonium compounds are however the alkyl benzyl dimethyl ammonium chlorides and mixtures thereof. e.g. that known generically as 'Benzalkonium chloride'. 'Benzalkonium chloride' may be used at a concentration of from 0.005 to 0.10 preferably 0.005 to 0.05, e.g. about 0.01% w/v and may optionally be used in combination with 0.2 to 2.0, e.g. 0.4% w/v of 2-phenylethanol (BPC 1963). 'Benzaolkonium chloride' and 2-phenylethanol have been found to have a synergistic effect, particularly against Pseudomonas aeruginosa, when used in combination with the disodium salt of ethylenediamine tetra acetic acid.

The solution composition may also contain conventional excipients, e.g. sodium chloride, dextrose or mannitol, and buffers, e.g. sodium dihydrogen orthophosphate (sodium acid phosphate BP), disodium hydrogen phosphate (sodium phosphate BP) sodium citrate/citric acid, and boric acid/sodium borate. The proportion and concentration of excipients and buffers may be varied within fairly wide ranges, provided the resulting solution is stable and non-irritant when applied to the appropriate tissues. For maximum stability the preferred pH is from 4.5 to 6.9, preferably 5.5 to 6.9, eg 6.0 to 6.9 with minimal buffering to avoid tissue irritation. The maximum total concentration of excipients and buffers is preferably less than 5% w/v and more preferably less than 2% w.v.

The composition may also contain additives designed to increase the viscosity and/or to prolong the action of the active ingredient.

Suitable additives include cellulosic compounds, e.g. methyl cellulose, hydroxypropylmethyl cellulose, hydroxypropylethyl cellulose, hydroxyethyl cellulose and ethylhydroxyethyl cellulose; polyvinyl alcohols; gelatin; polyvinylpyrolidone; polyethylene glycols; propylene glycol and glycerin.

Combinations of additives may be used if desired.

The concentration of the additives in the solution may be in the range 0.25 to 5% w/v and is preferably in the range 0.5 to 1.5% w/v.

The additives may of course be present in addition to any chelating or sequestering agents, preservatives, excipients, buffers, additional therapeutically useful compounds.

The aqueous composition, when it does not contain a preservative, may be made up using conventional techniques, e.g. by dissolving the chelating or sequestering agent (if included) in freshly distilled water, adding the excipients, buffers etc. to the aqueous solution of chelating or sequestering agent, adding the active ingredient to the resulting solution, stirring, filtering and then sterilising the composition by autoclaving, for example at a temperature of 115°C for 30 minutes. The autoclaving of the product may, if desired, be omitted when the composition is produced by sterile filtration into a previously sterilised container under aseptic conditions.

Aqueous compositions which contain preservatives may be made up by mixing two aqueous solutions of equal volumes one containing twice the desired final concentration of the active ingredient, chelating or sequestering agent, and other additives, and the other containing twice the desired final concentration of the preservative. The resulting mixture may then be filtered under sterile conditions.

The components of the composition should be as free as possible from 'metal ions' may be removed from the components of the composition by conventional means, e.g. by ion exchange.

Compositions for administration by nebuliser may be put up in single dose containers, e.g. ampules, containing from 0.5 to 5ml, e.g. 2ml, of solution.

The unpreserved compositions for administration to the eye may be put up in single application containers containing from 0.3 to 0.7 ml of solution and the preserved compositions may be put up in multi-dose (plastics, e.g. polyethylene, or glass) packs containing 5 to 20, preferably 7.5 or 17.5ml of solution.

We also provide solutions suitable for injection, e.g. sub-conjunctivally, preferably sterile unpreserved solutions which are substantially free of particulate material.

We further provide solutions for administration to the nose.

Whether a solution is substantially clear will be readily determinable by those skilled in the art, and will depend on the number, size and type of particles in the solution. Thus large particles are less acceptable than are particles of less than 50 μm diameter. We prefer the solutions of the invention to contain less than 20, preferably less than 16 and more preferably less than 12 particles per 8 ml of solution when the solution is viewed in a particle free container under the magnifier of a polarised light viewer. Desirably the solution contains less than 4 such particles per 8ml.

Compositions according to the invention possess the advantage that they are both palliative and prophylactic, are more effective, produce less side effects, can be used at lower doses, can be administered directly to the site of the allergy, e.g. by inhalation, are longer acting, are less likely to precipitate during nebulisation, are more stable, are synergistic, cause better patient compliance or possess other desirable properties as compared to certain mucolytic agents when used on their own, sodium cromoglycate when used on its own, or certain other mixtures when tested in relevant pharmacological models.

The invention is illustrated by the following Example.

Example 1

Solution for administration by nebuliser

| | |
|---|---|
| Sodium Cromoglycate, BP | 100g |
| N-acetyl-1-cysteine | 500g |
| Sodium edetate | 5g |
| Purified Water, BP to 10 litres | |

Method

A first solution is prepared by dissolving the N-acetyl-1-cysteine in approximately 4 litres of Purified Water, BP with stirring and, if necessary, gentle warming under a nitrogen atmosphere.

The pH of the solution is adjusted to 6.5 by the addition of sodium hydroxide solution.

A second solution is prepared by dissolving the Sodium Cromoglycate BP and then sodium edetate in approximately 4 litres of Purified Water BP with stirring under nitrogen.

The second solution is added to the first solution, the pH readjusted if necessary to 6.5 and the solution made up to 10 litres using Purified Water.

The solution may then be sterile filled in suitable containers, e.g. 2 ml glass ampoules

**Claims**

1. A pharmaceutical composition comprising an aqueous solution of from 0.6 to 6% w/v sodium cromoglycate and a mucolytic agent.

2. A composition according to Claim 1, comprising from 0.75 to 5% w/v sodium cromoglycate.

3. A composition according to Claim 1 or Claim 2, comprising from 0.8 to 4% w/v sodium cromoglycate.

4. A composition according to any one of the preceding claims wherein the mucolytic agent is N-acetyl-1-cysteine or a pharmaceutically acceptable salt thereof.

5. A composition according to Claim 4, comprising from 1 to 9% w/v N-acetyl-1-cysteine, measured as the free acid.

6. A composition according to any one of the preceding Claims, comprising an effective amount of a pharmaceutically acceptable chelating or sequestering agent.

7. A composition according to Claim 6, comprising from 0.005 to 0.1% w/v of the disodium salt of ethylenediaminetetraacetic acid.

8. A composition according to any one of the preceding claims having a pH of from 4.5 to 6.9.

9. A composition according to Claim 1, comprising 1.0% w/v sodium cromoglycate, 5% w/v N-acetyl-1-cysteine, 0.05% w/v sodium edetate and having a pH of from 6.0 to 6.9.

10. A method of making a composition according to Claim 6 and Claim 8, which comprises mixing a first solution containing the sodium cromoglycate and the chelating or sequestering agent with a second solution containing the mucolytic agent and adjusting the pH by addition of base.

**Revendications**

1. Composition pharmaceutique comprenant une solution aqueuse de 0,6 à 6% p/v de cromoglycate sodique et un agent mucolytique.

**EP 0 212 927 B1**

2. Composition suivant la revendication 1, comprenant 0,75 à 5% p/v de cromoglycate sodique.

3. Composition suivant la revendication 1 ou 2, comprenant 0,8 à 4% p/v de cromoglycate sodique.

4. Composition suivant l'une quelconque des revendications précédentes, dans laquelle l'agent mucolytique est la N-acétyl-1-cystéine ou un sel pharmaceutiquement acceptable de celle-ci.

5. Composition suivant la revendication 4, comprenant 1 à 9% p/v de N-acétyl-1-cystéine, mesurée en acide libre.

6. Composition suivant l'une quelconque des revendications précédentes, comprenant une quantité efficace d'un agent chélatant ou séquestrant pharmaceutiquement acceptable.

7. Composition suivant la revendication 6, comprenant 0,005 à 0,1% p/v du sel disodique de l'acide éthylènediaminetétraacétique.

8. Composition suivant l'une quelconque des revendications précédentes, ayant un pH de 4,5 à 6,9.

9. Composition suivant la revendication 1, comprenant 1,0% de p/v de cromoglycate sodique, 5% p/v de N-acétyl-1-cystéine et 0,05% p/v d'édétate sodique et ayant un pH de 6,0 à 6,9.

10. Procédé de préparation d'une composition suivant les revendications 6 et 8, qui comprend le mélange d'une première solution contenant le cromoglycate sodique et l'agent chélatant ou séquestrant avec une deuxième solution contenant l'agent mucolytique et l'ajustement du pH par addition d'une base.

**Patentansprüche**

1. Pharmazeutische Zusammensetzung, umfassend eine wässerige Lösung von 0,6 bis 6 % G/V Natriumcromoglycat und ein schleimlösendes Mittel.

2. Zusammensetzung nach Anspruch 1, umfassend 0,75 bis 5 % G/V Natriumcromoglycat.

3. Zusammensetzung nach Anspruch 1 oder 2, umfassend 0,8 bis 4 % G/V Natriumcromoglycat.

4. Zusammensetzung nach einem der vorhergehenden Ansprüchen, worin das schleimlösende Mittel N-Acetyl-1-cystein oder ein pharmazeutisch annehmbares Salz hievon ist.

5. Zusammensetzung gemäß Anspruch 4, umfassend 1 bis 9 % G/V N-Acetyl-1-cystein, gemessen als freie Säure.

6. Zusammensetzung nach einem der vorhergehenden Ansprüchen, umfassend eine wirksame Menge eines pharmazeutisch annehmbaren chelat- oder komplexbildenden Mittels.

7. Zusammensetzung gemäß Anspruch 6, umfassend 0,005 bis 0,1 % G/V des Dinatriumsalzes von Ethylendiamintetraessigsäure.

8. Zusammensetzung nach einem der vorhergehenden Ansprüchen mit einem pH von 4,5 bis 6,9.

9. Zusammensetzung gemäß Anspruch 1, umfassend 1,0 % G/V Natriumcromoglycat, 5 % G/V N-Acetyl-1-cystein, 0,05 % G/V Natriumedeteat, die einen pH von 6,0 bis 6,9 aufweist.

10. Verfahren zur Herstellung einer Zusammensetzung gemäß Anspruch 6 und 8, umfassend das Mischen einer ersten Lösung, die das Natriumcromoglycat und das chelat- oder komplexbildende Mittel enthält, mit einer zweiten Lösung, die das schleimlösende Mittel enthält, und Einstellen des pH durch Zugabe von Base.